# EUROPEAN PATENT APPLICATION

(11) **EP 4 682 264 A1**
(43) Date of publication of application: **21.01.2026**
(21) Application number: 24770951.2
(22) Date of filing: 13.03.2024
(51) Int. Cl.: C12Q 1/02, A61K 31/336, A61P 31/12, C12Q 1/37

(54) **ASSAY SYSTEM FOR DEVELOPING HONEYBEE ANTIVIRAL AGENT USING INHIBITION OF VIRUS-DERIVED PROTEASE AS INDICATOR**

(30) Priority: 13.03.2023 JP 2023038426
(71) Applicant: Toyama Prefectural University, Toyama, 939-0398 (JP); National University Corporation Hokkaido University, Hokkaido 060-0808 (JP)
(72) Inventor: KAMAKURA Masaki, Imizu-shi, Toyama 939-0398 (JP); NAKAOKA Shinji, Sapporo-shi, Hokkaido 060-0808 (JP)
(74) Representative: Dehns
(86) International application number: PCT/JP2024/009906
(87) International publication number: WO 2024/190848

(57) **Abstract**

[Problem] Provided are a screening method for a candidate compound that may serve as a prophylactic or therapeutic agent to suppress individual honey bee mortality caused by viral infection, as well as a prophylactic or therapeutic agent that contributes to the suppression of mass mortality of honey bees resulting from viral infection.

[Solution] The cause of mass mortality of honey bees has been identified as intestinal tract damage caused by a protease derived from a virus transmitted by mites, and it was found that individual mortality and intestinal tract damage in virus-infected groups can be suppressed by administering dsRNA of the virus. Furthermore, it was found that individual mortality in virus-infected groups can be suppressed by inhibiting the virus-derived protease through administration of E-64 ([(2S,3S)-3-carboxyoxirane-2-carbonyl]-L-leucine (4-guanidinobutyl)amide hemihydrate).

## Description

### Technical Field

The present invention relates to a prophylactic and/or therapeutic agent against a honey bee disease that is caused by intestinal tract damage in honey bees and that leads to individual honey bee mortality, as well as a screening method for the same.

This application claims priority to Japanese Patent Application No. 2023-038426, which is incorporated herein by reference.

Honey bees are agricultural insects necessary for pollination of crops. Honey bees are responsible for pollinating most fruits, many vegetables, seed and seedling production, forage crops for livestock, and other crops. Since pollination is essential for their production, the presence of honey bees is indispensable at agricultural production sites.

In recent years, mass mortality of honey bees has occurred worldwide due to outbreaks of *Varroa destructor,* a parasitic mite of honey bees. *Varroa destructor* has been identified as the primary cause of honey bee colony mortality reported by beekeepers in the United States in recent years (Non-Patent Document 1). *Varroa destructor* has spread from Asia to many other parts of the world through the human-mediated movement of honey bees, and if this situation is left unaddressed, the production of many agricultural products, including vegetables and fruits, could become impossible. The mass mortality of honey bees, which play the role of pollinators, poses a major problem for the agriculture and apiculture industries, and is also an important issue from the perspective of biodiversity.

Recently, it was reported that bacteria genetically modified to have RNAi functionality, when administered to honey bees, could reduce viruses infecting honey bees and decrease the population of parasitic mites (Non-Patent Document 2). However, while this report demonstrates that it is technically possible to decrease viruses and mite populations, the technique cannot be easily applied in actual beekeeping settings because it deals with a genetically modified organism.

Solving the problem of mass mortality of honey bees is tied to the stable supply of agricultural products and has become an important issue in the field of agriculture. However, until now, the pathogens responsible for mass mortality of honey bees, the mechanisms by which these pathogens cause disease, and their remedies have not been clarified at all.

### Prior Art Documents

### Non-Patent Documents

Non-Patent Document 1: Steinhauer, N., et. al., "United States honey bee colony losses 2022-23: Preliminary results from the Bee Informed Partnership", [online], June 22, 2023, Bee Informed Partnership, [retrieved on March 10, 2024], Internet <https://beeinformed.org/2023/06/22/united-states-honey-bee-colony-losses-2022-23-preliminary-results-from-the-bee-informed-partnership/>
Non-Patent Document 2: Leonard, S. P., et. al., Science. 2020 Jan 31; 367(6477): 573-576

### Disclosure of Invention

### Problem to be Solved by the Invention

An object of the present invention is to provide a screening method for a candidate compound that may serve as a prophylactic or therapeutic agent to suppress individual honey bee mortality caused by viral infection, as well as a prophylactic or therapeutic agent that contributes to the suppression of mass mortality of honey bees resulting from viral infection.

### Means for Solving Problem

As a result of in-depth research conducted in order to address the above-described issues, the inventors of the present invention identified that the cause of mass mortality of honey bees is intestinal tract damage due to a protease derived from a virus transmitted by mites, and found that individual mortality and intestinal tract damage in virus-infected groups can be suppressed by administering dsRNA of the virus. Furthermore, the inventors of the present invention found that individual mortality in virus-infected groups can be suppressed by inhibiting the virus-derived protease through the administration of E-64 ([(2S,3S)-3-carboxyoxirane-2-carbonyl]-L-leucine (4-guanidinobutyl)amide hemihydrate), and thus achieved the present invention.

Specifically, the present invention provides the following.
1. A screening method for a prophylactic and/or therapeutic agent against a honey bee disease that is caused by intestinal tract damage in honey bees and that leads to individual mortality, using inhibitory activity against a virus-derived protease as an indicator.
2. The screening method according to clause 1, wherein the honey bees are honey bees infected with a virus transmitted by a mite.
3. The screening method according to clause 1 or 2, wherein the protease is a cysteine protease.
4. The screening method according to clause 1 or 2, wherein the virus is Israeli acute paralysis virus (IAPV) or deformed wing virus (DWV).
5. The screening method according to clause 1, further including use of individual honey bee mortality and/or honey bee intestinal tract regeneration as an indicator.
6. A prophylactic and/or therapeutic agent against a honey bee disease that is caused by intestinal tract damage in honey bees and that leads to individual honey bee mortality, the agent containing a substance that inhibits activity of a virus-derived protease.
7. The prophylactic and/or therapeutic agent according to clause 6,
   wherein the substance that inhibits activity of the virus-derived protease is E-64 and/or an analog thereof represented by the general formula (I) below: 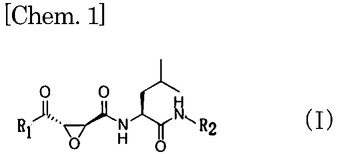
   where
   R₁ represents OH, an alkoxy group that may be substituted, or an amino group that may be substituted; and
   R₂ represents an alkyl group that may be substituted, or a heterocycloalkyl that forms a ring via N to which R₂ is bonded, and that may be substituted.
8. The prophylactic and/or therapeutic agent according to clause 6, wherein the substance that inhibits activity of the virus-derived protease is a calpain inhibitor.

### Effects of the Invention

According to the present invention, individual honey bee mortality caused by viral infection can be suppressed by inhibiting the activity of the virus-derived protease, and the substance that inhibits the activity of the virus-derived protease can be used as an active ingredient in the prophylactic or therapeutic agent against a honey bee disease that is caused by viral infection and that leads to individual honey bee mortality. According to the present invention, it is possible to provide a screening method for a candidate compound that may serve as a prophylactic or therapeutic agent against a honey bee disease that is caused by viral infection and that leads to individual honey bee mortality, and it is possible to provide a prophylactic or therapeutic agent against a honey bee disease that leads to individual honey bee mortality, based on a novel mechanism of action involving the suppression of intestinal tract damage through inhibition of the activity of a virus-derived protease.

### Brief Description of Drawings

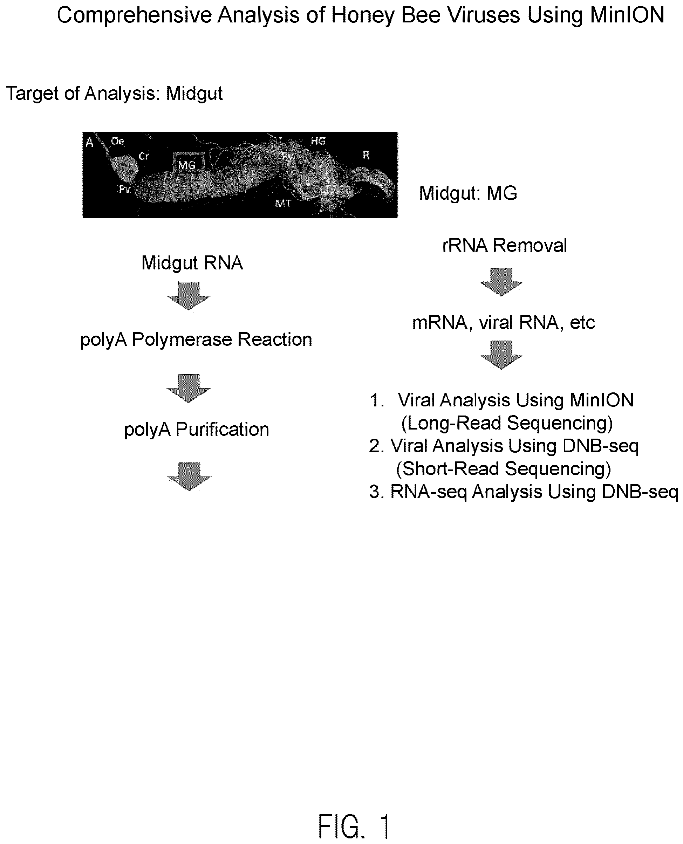

[FIG. 1] FIG. 1 is a schematic diagram of the sample preparation procedure for MinION analysis. (Example 1)

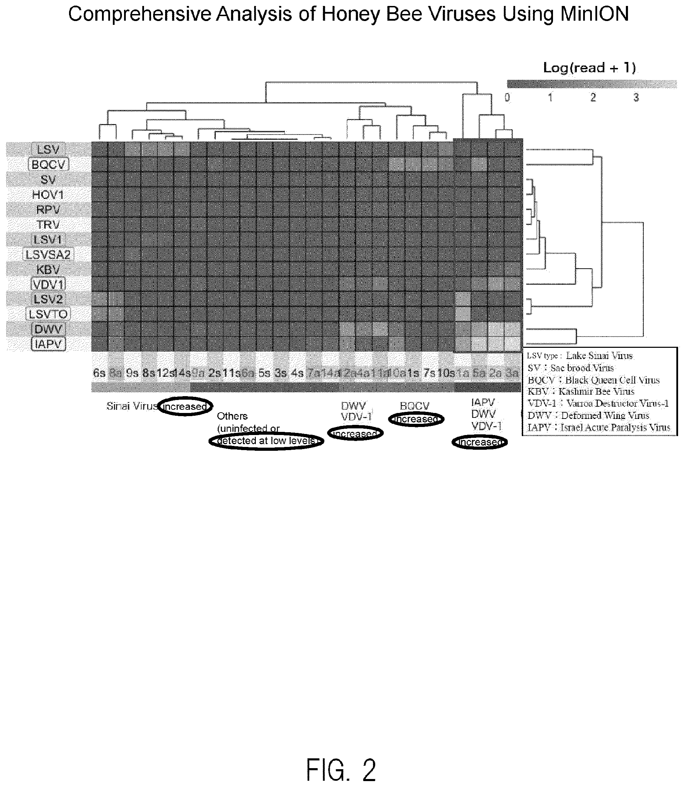

[FIG. 2] FIG. 2 is a heatmap showing the results of calculating quantitative values of each viral load in the MinION analysis. (Example 1)

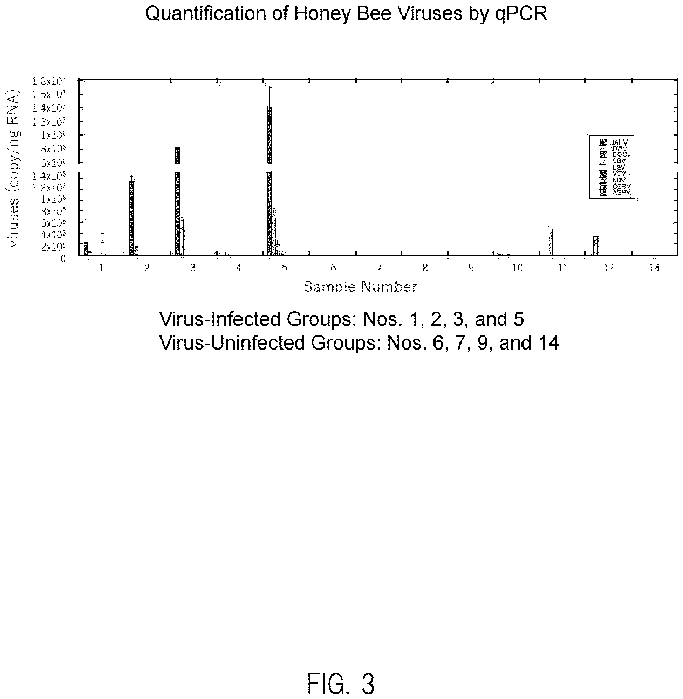

[FIG. 3] FIG. 3 shows the results of RT-PCR-based quantification of honey bee viruses. The horizontal axis indicates the sample number (honey bee population No.), and the vertical axis indicates the number of viral copies per ng of RNA. (Example 2)

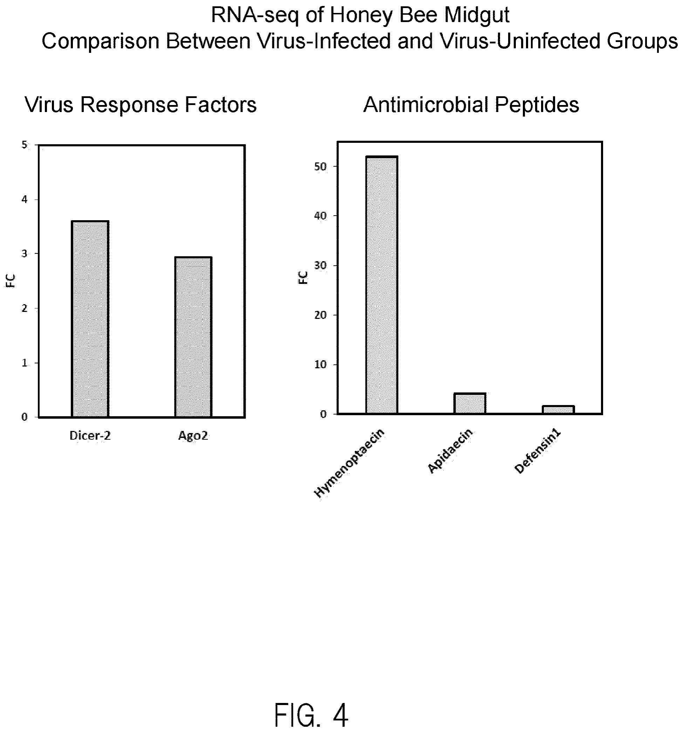

[FIG. 4] FIG. 4 shows the results of quantification of viral response factors and antimicrobial peptides in honey bees. The horizontal axis indicates the gene name, and the vertical axis indicates the expression level ratio (Fold Change; FC). (Example 3)

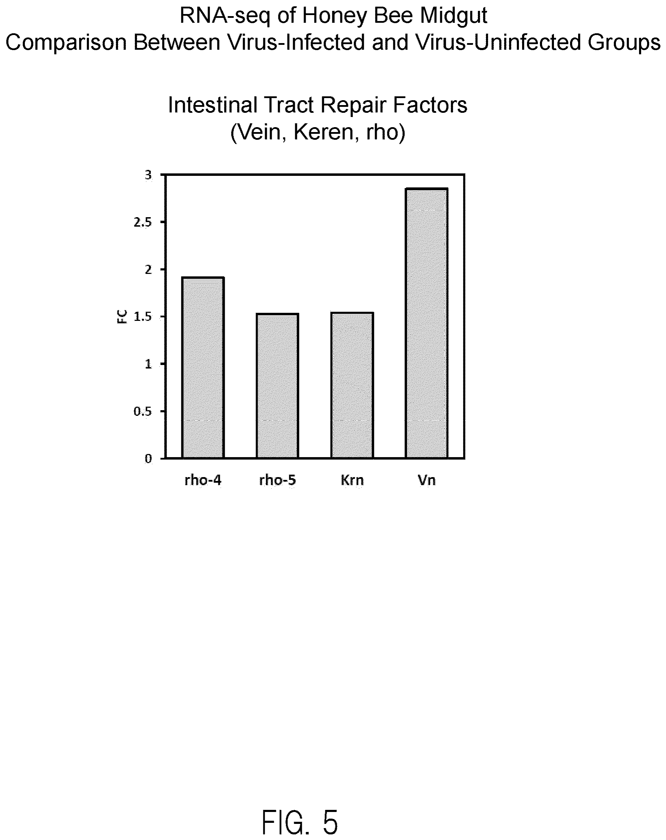

[FIG. 5] FIG. 5 shows the results of quantification of intestinal tract repair factors in honey bees. The horizontal axis indicates the gene name, and the vertical axis indicates the expression level ratio (Fold Change; FC). (Example 3)

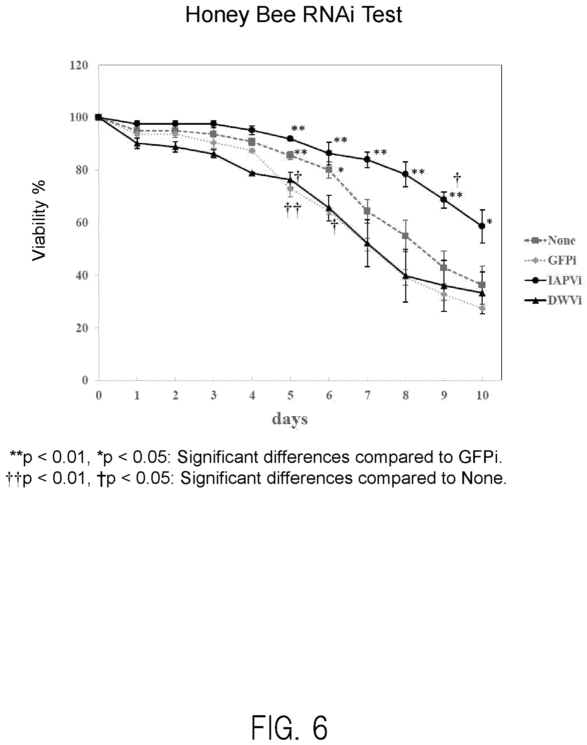

[FIG. 6] FIG. 6 shows the results of RNAi tests for viruses in honey bees. The horizontal axis indicates the number of days elapsed from the start of administration, and the vertical axis indicates the viability, which is the number of individuals on each day expressed as a percentage, with the number of individuals on day 0 set to 100%. (Example 4)

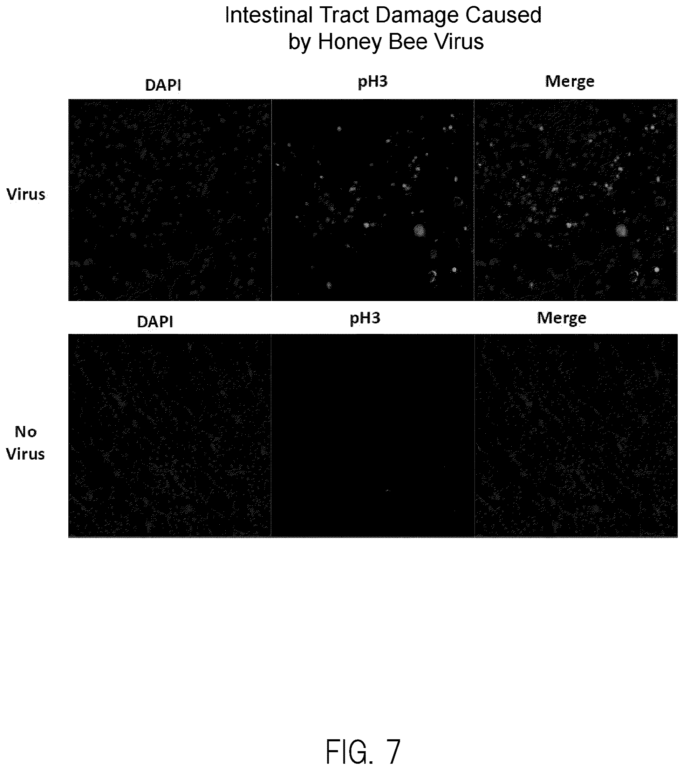

[FIG. 7] FIG. 7 shows the detection results of pH3 antibody-positive cells in honey bees. Microscopic images from the midgut of virus-infected and virus-uninfected groups are shown, including DAPI-stained, pH3-immunostained, and merged (Merge) images. (Example 5)

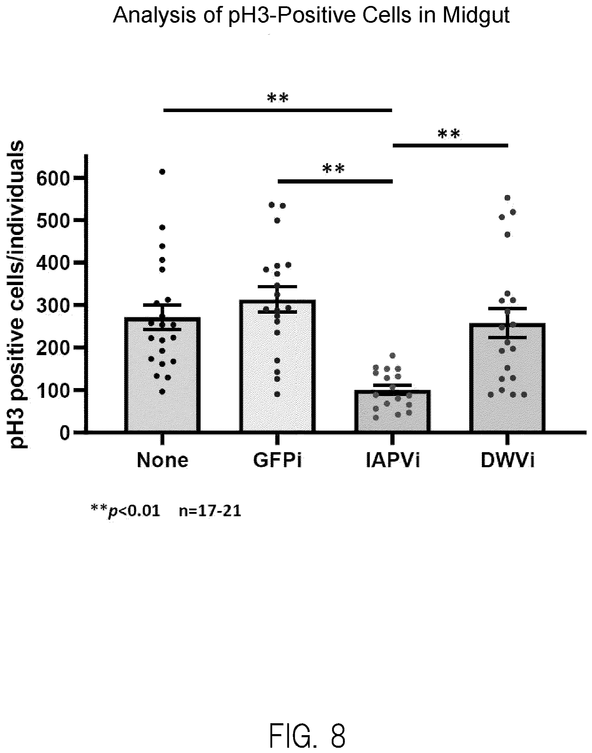

[FIG. 8] FIG. 8 shows the analysis results of pH3 antibody-positive cells after dsRNA administration. The horizontal axis indicates administered groups: "None" denotes a control group, "GFPi" denotes a group to which GFP dsRNA was administered, "IAPVi" denotes a group to which IAPV dsRNA was administered, and "DWVi" denotes a group to which DWV dsRNA was administered. The vertical axis indicates the number of pH3 antibody-positive cells in the midgut per individual. (Example 5)

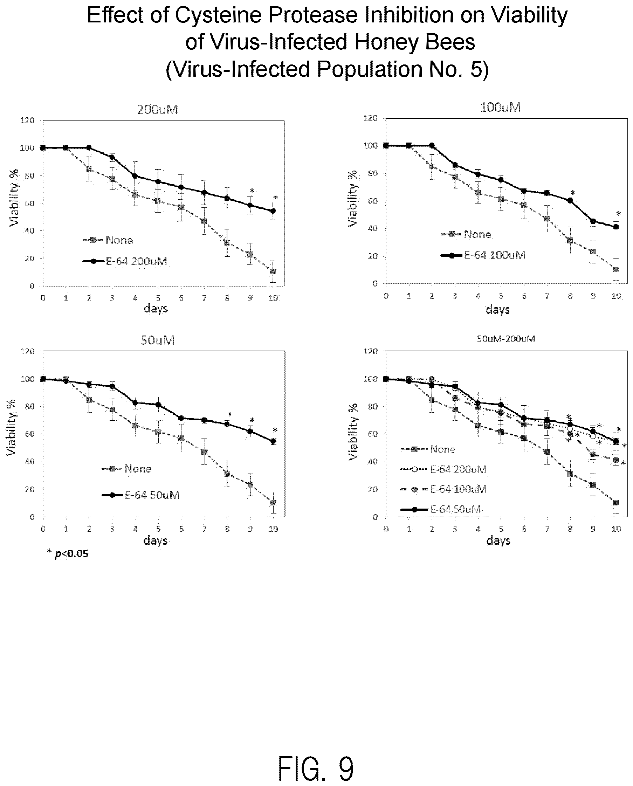

[FIG. 9] FIG. 9 shows the results of E-64 administration in suppressing individual honey bee mortality in a virus-infected group No. 5. The horizontal axis indicates the number of days elapsed from the start of administration, and the vertical axis indicates the viability, which is the number of individuals on each day expressed as a percentage, with the number of individuals on day 0 set to 100%. (Example 6)

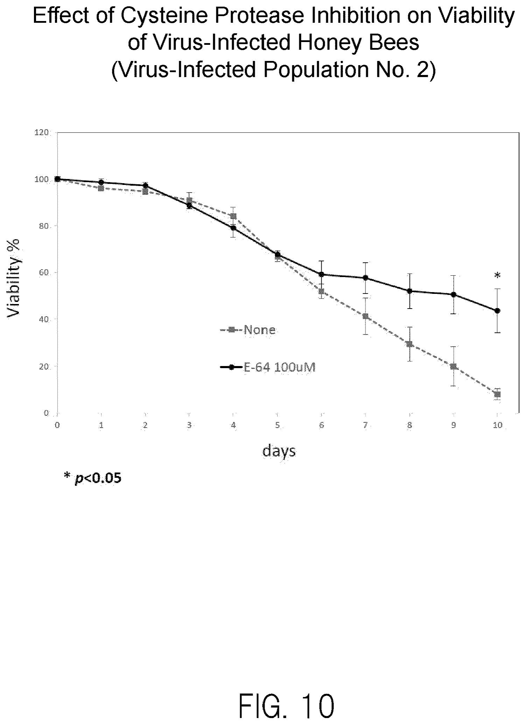

[FIG. 10] FIG. 10 shows the results of E-64 administration in suppressing individual honey bee mortality in a virus-infected group No. 2. The horizontal axis indicates the number of days elapsed from the start of administration, and the vertical axis indicates the viability, which is the number of individuals on each day expressed as a percentage, with the number of individuals on day 0 set to 100%. (Example 6)

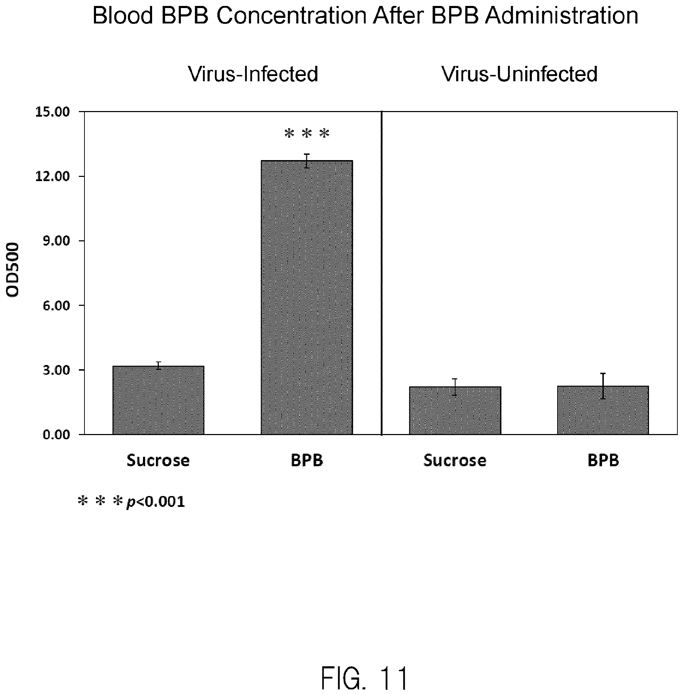

[FIG. 11] FIG. 11 shows the results of a BPB administration test. In each of virus-infected and virus-uninfected groups, "Sucrose" and "BPB" denote the results of samples collected after Sucrose administration and BPB administration, respectively. The vertical axis indicates the absorbance measured at 500 nm. (Example 7)

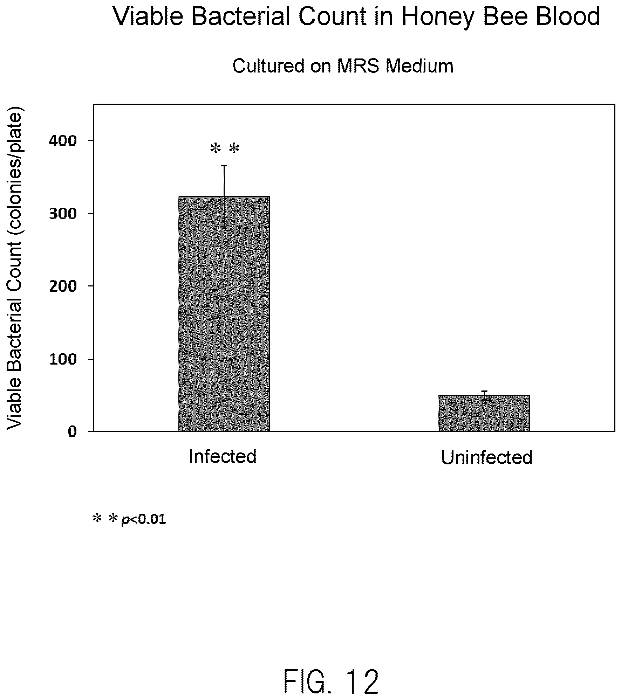

[FIG. 12] FIG. 12 shows the results of viable bacterial count measurements in the blood of honey bees. "Infected" denotes the result of a virus-infected group, and "Uninfected" denotes the result of a virus-uninfected group. The vertical axis indicates the number of viable bacteria (colony/a plate). (Example 8)

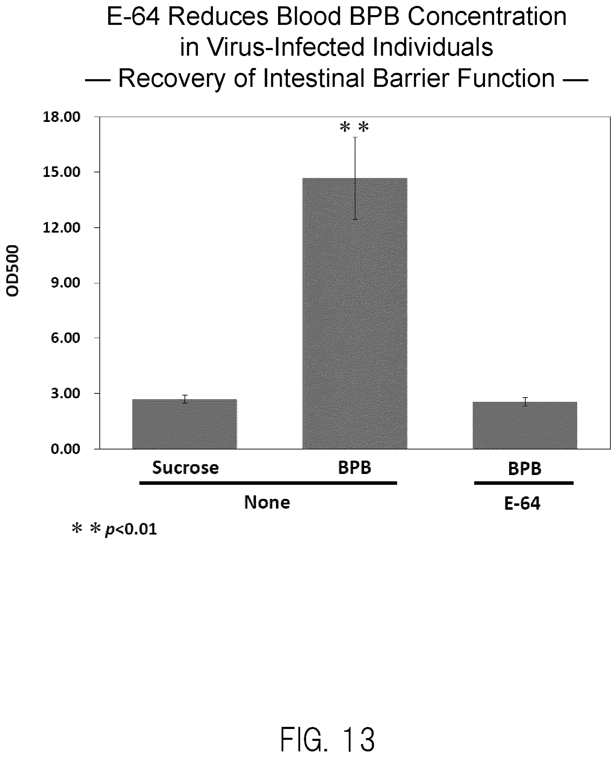

[FIG. 13] FIG. 13 shows the results of a confirmation experiment on the recovery from intestinal tract damage in honey bees, achieved through administration of E-64. "None" denotes a control group, "E-64" denotes a group to which E-64 was administered, and "Sucrose" and "BPB" denote the results of samples collected after Sucrose administration and BPB administration, respectively. The vertical axis indicates the absorbance measured at 500 nm. (Example 9)

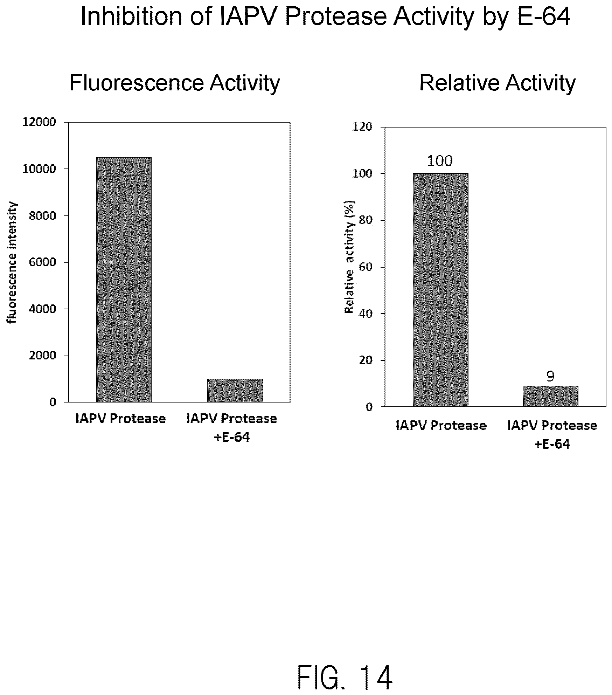

[FIG. 14] FIG. 14 shows the results of protease activity measurements for IAPV protease. For both fluorescence activity and relative activity, "IAPV Protease" denotes the result of the enzymatic reaction of IAPV protease measured in the absence of E-64, and "IAPV Protease + E-64" denotes the result of the enzymatic reaction of IAPV protease measured in the presence of E-64. The vertical axis of the left graph indicates fluorescence intensity. The vertical axis of the right graph indicates the relative value (relative activity) of IAPV protease activity, calculated based on fluorescence intensity, with the fluorescence intensity measured from the enzymatic reaction of IAPV protease in the absence of E-64 set to 100%. (Example 12)

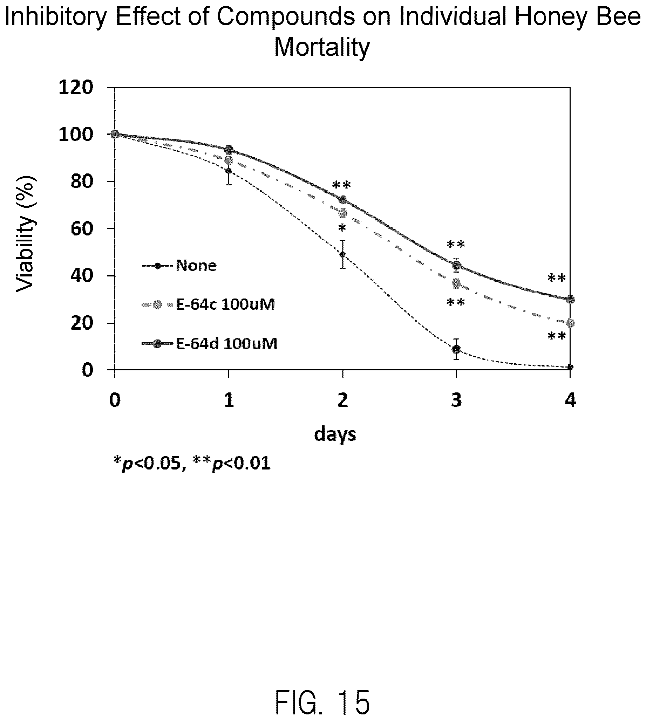

[FIG. 15] FIG. 15 shows the results of analysis of the effects of E-64c and E-64d on individual honey bee mortality. The horizontal axis indicates the number of days elapsed from the start of administration, and the vertical axis indicates the viability, which is the number of individuals on each day expressed as a percentage, with the number of individuals on day 0 set to 100%. (Example 13)

### Description of the Invention

The present invention is based on the following findings: high levels of viruses, particularly Israeli acute paralysis virus (IAPV), transmitted by mites have been detected in colonies of honey bees that experienced mass mortality; intestinal tract damage has been observed in virus-infected groups; administration of viral dsRNA suppresses individual mortality and midgut damage; and administration of viral dsRNA suppresses individual mortality and intestinal tract damage in virus-infected groups. From these findings, the present invention identifies a virus transmitted by mites as the pathogen responsible for mass mortality of honey bees, and, for the first time, reveals the molecular mechanism by which intestinal tract damage due to a virus-derived protease induces mass mortality of honey bees. A screening method of the present invention, based on these findings, leads to the development of a novel pharmaceutical agent for honey bees through a completely different approach from conventional ones. Furthermore, the present invention is based on the finding that administration of E-64 ([(2S,3S)-3-carboxyoxirane-2-carbonyl]- L-leucine (4-guanidinobutyl)amide hemihydrate) suppresses individual mortality in virus-infected groups.

The present invention provides a screening method for a substance effective as a prophylactic and/or therapeutic agent against a honey bee disease that is caused by intestinal tract damage in honey bees and that leads to individual honey bee mortality, the method comprising using inhibitory activity against a virus-derived protease as an indicator, and substances that inhibit the activity of the protease as candidate compounds.

The term "inhibitory activity against a virus-derived protease" refers to the activity that inhibits (suppresses) the activity of a virus-derived protease. This activity can be evaluated, for example, by assessing substrate degradation or the like by the virus-derived protease. Furthermore, this activity can be evaluated by assessing an event resulting from the inhibition of the activity of the virus-derived protease in the presence of a test substance.

Examples of the event resulting from the inhibition of the activity of the virus-derived protease include suppression of individual mortality (i.e., improved viability) of virus-infected honey bees, recovery from intestinal tract damage, suppression of intestinal tract damage, reduction in intestinal tract regeneration due to intestinal tract damage, and the like. Suppression of intestinal tract damage and reduction in intestinal tract regeneration can be evaluated based on the number of cells positive for anti-phospho-histone H3 antibody (pH3 antibody). More specifically, in intestinal tracts damaged by virus-derived proteases, repair (regeneration) of the damaged intestinal tracts occurs, during which mitosis can be observed in the cells. Since the pH3 antibody recognizes phospho-histone H3, which is specific to mitosis, mitotic cells can be detected as positive images using the pH3 antibody. When intestinal tract damage is suppressed, the number of mitotic cells observed during the regeneration of the damaged intestinal tract decreases, resulting in a reduced number of pH3 antibody-positive cells in the midgut of each honey bee. Recovery from and suppression of intestinal tract damage can be evaluated based on the amount of Bromophenol Blue (BPB) in the blood of honey bees after BPB administration. Since BPB is absorbed into the body as a result of intestinal tract damage, the amount of BPB detected in the blood increases after BPB administration, and as a result of recovery from intestinal tract damage, the amount of BPB detected in the blood after BPB administration decreases. Suppression of intestinal tract damage results in suppression of an increase in the amount of BPB detected in the blood after BPB administration.

Accordingly, the screening method of the present invention enables a more multifaceted evaluation of the inhibitory activity of a test substance against a virus-derived protease, not only by assessing its inhibitory activity against a virus-derived protease but also by assessing such an event as an indicator.

The test substance to be used in the screening method of the present invention is not particularly limited, and may be any known or novel compound. For example, the test substance may be a biologically produced substance, a chemically synthesized compound, or the like. More specifically, examples include nucleic acids, nucleic acid analogs, carbohydrates, lipids, proteins, peptides, antibodies, antibody fragments, organic low-molecular-weight compounds, compound libraries, and the like.

In one embodiment, the screening method of the present invention includes the following steps:
(A) a step of bringing a virus-derived protease into contact with a test substance;
(B) a step of measuring the activity of the virus-derived protease that has been brought into contact with the test substance; and
(C) a step of comparing the measured activity of the virus-derived protease with the activity of the virus-derived protease that has not been brought into contact with the test substance, and selecting the test substance as a candidate substance for a prophylactic and/or therapeutic agent against a honey bee disease that is caused by intestinal tract damage in honey bees and that leads to individual honey bee mortality, if the comparison shows that the activity of the virus-derived protease that has been brought into contact with the test substance is lower than the activity of the virus-derived protease that has not been brought into contact with the test substance.

In the step (A) of the above method, a virus-derived protease and a test substance are placed under conditions that allow contact. As the virus-derived protease, a protein synthesized using a known protein synthesis system can be used. For example, an *Escherichia coli* protein synthesis system, an insect cell protein synthesis system, an animal cell protein synthesis system, a cell-free protein synthesis system, and the like can be used.

In the step (B) of the above method, the activity of the virus-derived protease can be measured using a known or novel method of protease activity measurement. For example, the activity can be measured using a protease activity measurement method that utilizes the degradation of a substrate (e.g., casein, a synthetic substrate, etc.). In an example of the protease activity measurement method that utilizes the degradation of a substrate, the activity of a virus-derived protease can be measured by adding the virus-derived protease to the substrate in the presence of a test substance, and then quantifying the undegraded portion and/or the degraded portion of the substrate after a certain period of time has elapsed. The quantification of the undegraded portion and/or the degraded portion of the substrate can be performed using, for example, a method that employs a fluorescently labeled substrate, electrophoresis, absorbance measurement, or the like. When a method utilizing the degradation of a substrate is employed in the step (B), the contact between the virus-derived protease and the test substance in the step (A) may be carried out in a solution containing the protease substrate, or alternatively, the virus-derived protease, after being brought into contact with the test substance, may be further brought into contact with the substrate in a separate solvent. The greater the amount of the undegraded portion of the substrate, the lower the protease activity, while the greater the amount of a substrate degradation product, the higher the protease activity.

In the step (C) of the above method, the activity of the virus-derived protease that has been brought into contact with the test substance is compared with the activity of a control virus-derived protease that has not been brought into contact with the test substance. Preferably, the comparison of protease activity is based on the presence or absence of a significant difference.

In one embodiment, the screening method of the present invention includes, in addition to the above-described steps (A) to (C), the following steps (a) and (b), steps (a) and (c), or steps (a), (b), and (c):
(a) a step of administering the test substance to a honey bee population and rearing the honey bees;
(b) a step of measuring the viability of the honey bee population, comparing the measured viability with the viability of a control group that has been reared without administering the test substance, and selecting the test substance as a candidate substance for a prophylactic and/or therapeutic agent against a honey bee disease that is caused by intestinal tract damage in honey bees and that leads to individual honey bee mortality, if the comparison shows that the viability of the honey bee population that has been reared with administration of the test substance is higher than the viability of the control group without administration of the test substance; and
(c) a step of counting the number of cells positive for anti-phospho-histone H3 antibody (pH3 antibody) in the midgut of each individual honey bee in the honey bee population, comparing an average value of the counted numbers with an average value of the numbers of pH3 antibody-positive cells in the control group that has been reared without administration of the test substance, and selecting the test substance as a candidate substance for a prophylactic and/or therapeutic agent against a honey bee disease that is caused by intestinal tract damage in honey bees and that leads to individual honey bee mortality, if the comparison shows that the average values of the numbers of pH3 antibody-positive cells in the honey bee population that has been reared with administration of the test substance is lower than the average value of the numbers of pH3 antibody-positive cells in the control group that has been reared without administration of the test substance, or a step of administering Bromophenol Blue (BPB) to the honey bee population that has been reared with administration of the test substance and to the control group that has been reared without administration of the test substance, measuring the concentration of BPB in the blood following BPB administration, and selecting the test substance as a candidate substance for a prophylactic and/or therapeutic agent against a honey bee disease that is caused by intestinal tract damage in honey bees and that leads to individual honey bee mortality, if the increase in the concentration of BPB in the blood resulting from BPB administration is suppressed (reduced) compared to that in the control group.

In the step (a) of the above method, the number of individuals at the start of rearing in each of the groups, that is, the honey bee population reared with administration of the test substance and the control group reared without administration of the test substance, is not particularly limited. For example, the number may be 1 to 200 or 10 to 100, and is preferably 20 to 50, and more preferably 30 to 35. Preferably, the steps (a) to (c) are performed in a sterile environment. The temperature at which each group is reared is not particularly limited, but is preferably 15°C to 35°C, more preferably 20°C to 35°C, even more preferably 25°C to 35°C, and yet even more preferably 27°C to 33°C.

In the step (a) of the above method, the method of administering the test substance is not particularly limited; however, it is preferable that the test substance is incorporated into feed (e.g., a sugar-containing solution such as a sucrose solution) and provided to the honey bees *ad libitum.* The concentration of the test substance is not particularly limited; however, in the case of E-64 or an analog thereof or a calpain inhibitor, the concentration may be 1 to 1000 µM, and is preferably 10 to 500 µM, more preferably 10 to 300 µM, even more preferably 20 to 250 µM, yet even more preferably 50 to 200 µM, and yet even more preferably 50 to 150 µM. The honey bee population used in the step (a) is not particularly limited, but is preferably a honey bee population derived from a virus-infected colony.

The comparison of viability in the step (b) of the above method is performed after a certain period of time has elapsed from the start of administration in the step (a), and the elapsed period is not particularly limited; however, the comparison is preferably performed after 1 day or more, more preferably after 2 days or more, even more preferably after 3 days or more, and yet even more preferably after 4 days or more have elapsed from the start of administration. For example, the comparison may be performed after 1 to 30 days, after 1 to 20 days, after 1 to 15 days, after 2 to 14 days, after 3 to 13 days, after 4 to 12 days, after 5 to 11 days, after 6 to 10 days, after 7 to 10 days, after 8 to 10 days, after 9 to 10 days, or after 10 days from the start of administration. In the step (b), the number of times viability is measured may be only once; however, it is preferable that viability is measured at regular intervals. The measurement interval is not particularly limited, but may be, for example, once every 1 hour to 3 days, and is preferably once every 12 hours to 2 days, more preferably once every 18 to 36 hours, and even more preferably once per day. When viability is measured at regular intervals, the comparison of viability is preferably performed at the time when the viability of the control group, measured at regular intervals, has decreased to below 100%. For example, the comparison may be performed at the time when the viability of the control group has decreased to 90% or less, 80% or less, 70% or less, 60% or less, 50% or less, 40% or less, 30% or less, 20% or less, or 10% or less. Preferably, the comparison of viability is based on the presence or absence of a significant difference.

In the step (c), the counting of the number of pH3 antibody-positive cells in the midgut is performed after a certain period of time has elapsed from the start of administration in the step (a). The elapsed period is not particularly limited; however, the counting is preferably performed after 1 day or more, more preferably after 2 days or more, even more preferably after 3 days or more, and yet even more preferably after 4 days or more have elapsed from the start of administration. For example, the counting may be performed after 1 to 30 days, after 1 to 20 days, after 1 to 15 days, after 2 to 12 days, after 3 to 11 days, after 4 to 10 days, after 5 to 9 days, or after 6 to 8 days from the start of administration. In the step (c), the number of individuals used for counting the number of pH3 antibody-positive cells in the midgut is not particularly limited, and may include all surviving individuals in the honey bee population at the time of counting, or a portion thereof (e.g., 1% to 99%, 10% to 90%, 20% to 80%, 30% to 70%, 40% to 60%, or the like of all the surviving individuals). The timing of BPB administration in the step (c) is not particularly limited as long as it is after the start of administration in the step (a); however, for example, BPB administration may be performed when the viability of the control group, which has been reared without administration of the test substance, has decreased to 90% or less, 80% or less, 70% or less, 60% or less, 50% or less, 40% or less, 30% or less, 20% or less, or 10% or less, relative to the number of honey bees before rearing in the step (a), which is set to 100%.

The present invention provides a prophylactic and/or therapeutic agent against a honey bee disease that is caused by intestinal tract damage in honey bees and that leads to individual honey bee mortality, the agent containing a substance that inhibits the activity of a virus-derived protease.

The prophylactic and/or therapeutic agent of the present invention can be mixed into water and/or feed (e.g., a sugar-containing solution, such as a sucrose solution, or pollen, honey, artificial feed, or the like) so that the resulting mixture contains an effective amount of the substance that inhibits the activity of the virus-derived protease, and administered to honey bee through water intake, feeding, or the like. The effective amount of the substance that inhibits the activity of the virus-derived protease in water intake, feeding, or the like may be 1 to 1000 µM, and is preferably 10 to 500 µM, more preferably 10 to 300 µM, even more preferably 20 to 250 µM, yet even more preferably 50 to 200 µM, and yet even more preferably 50 to 150 µM, when the substance is E-64 or an analog thereof or a calpain inhibitor. The prophylactic and/or therapeutic agent of the present invention may also be used by spraying onto, immersion of, application to, and/or mixing with honey bees, hive boxes, nectar source plants for honey bees (including their growing environment such as soil), and/or a suitable material. The material may be, for example, installed in or around a hive box, and the agent can be administered by allowing honey bees to come into contact with this material.

The dosage form of the prophylactic and/or therapeutic agent of the present invention is not particularly limited, and the agent may be formulated into a liquid, suspension, wettable powder, emulsion, aerosol, powder, granule, tablet, or the like by mixing with a pharmaceutically acceptable carrier or additive. A preferable dosage form is a liquid. The production methods for these dosage forms are not particularly limited, and they can be formulated according to techniques available for general agrochemical formulations. The amount of the substance that inhibits the activity of the virus-derived protease in a formulation is not particularly limited, but may be, for example, 0.000000001 to 99 w/v%, 0.00000001 to 50 w/v%, 0.0000001 to 1 w/v%, 0.0000001 to 0.1 w/v%, 0.001 to 50 w/v%, 0.1 to 10 w/v%, or the like. Examples of the pharmaceutically acceptable carrier or additive include, but are not limited to, sugars such as sucrose, dextrin, and glucose; surfactants such as sodium lauryl sulfate, laurylamine acetate, and polyoxyethylene lauryl alcohol; lubricants such as talc and calcium stearate; stabilizers such as tocopherol, ascorbic acid, and vitamins; thickeners such as starch, xanthan gum, and polyvinylpyrrolidone; and the like.

The virus in the present invention is preferably a virus transmitted by a mite, and examples thereof include Israeli acute paralysis virus (IAPV), deformed wing virus (DWV), acute bee paralysis virus (ABPV), black queen cell virus (BQCV), chronic bee paralysis virus (CBPV), sacbrood virus (SBV), Varroa destructor virus-1 (VDV-1), Kashmir bee virus (KBV), Lake Sinai virus (LSV), and the like. Israeli acute paralysis virus or deformed wing virus is preferred, and Israeli acute paralysis virus is more preferred.

The virus-derived protease in the present invention is preferably a virus-derived cysteine protease, more preferably a cysteine protease derived from IAPV or DWV, and even more preferably a cysteine protease derived from IAPV, examples of which include proteases having the amino acid sequences shown in SEQ ID NOs: 51, 52, and 53, as well as proteases resulting from alterations based on mutations. For example, the virus-derived protease in the present invention may be a polypeptide in which 1 to 40, preferably 1 to 20, more preferably 1 to 10, even more preferably 1 to 5, and yet even more preferably 1 to 3 amino acid residues are deleted, added, inserted, or substituted within the amino acid sequence shown in SEQ ID NO: 51, 52, or 53, and which has protease activity. In addition, examples of a base sequence of a cDNA encoding the cysteine protease derived from IAPV include the base sequences shown in SEQ ID NOs: 54, 55, and 56, as well as base sequences resulting from alterations based on polymorphisms and mutations, and examples include those in which 1 to 50, preferably 1 to 20, more preferably 1 to 5, and even more preferably 1 to 3 bases are deleted, substituted, and/or added within the base sequence shown in SEQ ID NO: 54, 55, or 56. Furthermore, the base sequence of the cDNA encoding the cysteine protease derived from IAPV includes a variant consisting of a nucleotide sequence that has at least 50% identity to the base sequence shown in SEQ ID NO: 54, 55, or 56, for example, at least 60%, at least 70%, at least 80%, at least 85%, at least 90%, at least 95%, at least 97%, at least 98%, at least 99%, at least 99.5%, at least 99.7%, or at least 99.9% identity thereto. The base sequence identity can be determined using known algorithms, such as BLAST or FASTA. Furthermore, the base sequence of the cDNA encoding the cysteine protease derived from IAPV includes a variant consisting of a base sequence that hybridizes to the base sequence shown in SEQ ID NO: 54, 55, or 56 under stringent conditions. Examples of the stringent hybridization conditions include, for example, typical post-hybridization wash conditions such as "5×SSC, 1% SDS, 37°C", "1×SSC, 0.1% SDS, 37°C", "0.5×SSC, 0.1% SDS, 42°C", "5×SSC, 1% SDS, 65°C", and other similar conditions. In the present invention, a polypeptide can be determined to "have protease activity" if, upon incubation at 37°C for 1 hour, a solution containing the polypeptide and a substrate (casein or a synthetic substrate) dissolved in a solvent exhibits a higher amount of substrate degradation (i.e., a lower amount of undegraded substrate) compared to a control solution in which only the substrate is dissolved in the solvent.

There is no particular limitation on the substance that inhibits the activity of the cysteine protease derived from IAPV in the present invention; however, examples include organic low-molecular-weight compounds, nucleic acids, carbohydrates, lipids, proteins, peptides, and the like. Examples of the organic low-molecular-weight compounds include E-64 ([(2S,3S)-3-carboxyoxirane-2-carbonyl]-L-leucine (4-guanidinobutyl)amide), analogs of E-64, calpain inhibitors, and the like, and any of their hydrates, hemihydrates, and anhydrides, mixtures thereof, and the like may be used. Examples of the nucleic acids include siRNAs and antisense oligonucleotides that target a cysteine protease gene derived from IAPV, their expression vectors, and the like. As used herein, the term "siRNA" is an abbreviation for short interfering RNA, which is an RNA molecule having a double-stranded RNA region of approximately 10 to 40 bases (hereinafter sometimes referred to as dsRNA). An siRNA has the function of cleaving the mRNA of a target gene that has a sequence complementary to its antisense strand and thereby suppressing the expression of the target gene.

Examples of the analogs of E-64 in the present invention include E-64-c ([(2S,3S)-3-carboxyoxirane-2-carbonyl]-L-leucine (3-methylbutyl)amide, E-64-d ([(2S,3S)-3-ethoxycarbonyloxirane-2-carbonyl]-L-leucine (3-methylbutyl)amide), JPM-OEt ((2S,3S)-3-[[[(1S)-1-[[[2-(4-hydroxyphenyl)ethyl]amino]carbonyl]-3-methylbutyl]amino]carbonyl]-2-oxirane carboxylic acid ethyl ester), CA-074 ([(2S,3S)-3-propylcarbamoyloxirane-2-carbonyl] - L-isoleucyl- L-proline), CA-074Me ([(2S,3S)-3-propylcarbamoyloxirane-2-carbonyl]-L-isoleucyl-L-proline methyl ester), Ep-459, Ep-460, Ep-479, Ep-429, and the like. Ep-459, Ep-460, Ep-479, and Ep-429 have the following structures, respectively.

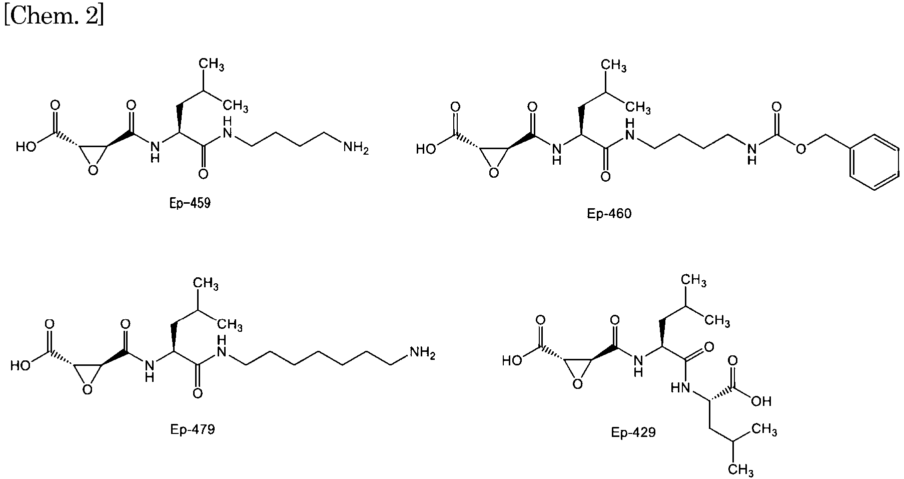

According to an embodiment of the present invention, the substance that inhibits the activity of the virus-derived protease is E-64 and/or an analog thereof and is represented by the general formula (I) below.

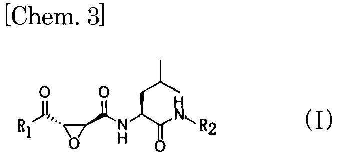

In the general formula (I),
R₁ represents OH, an alkoxy group that may be substituted, or an amino group that may be substituted; and
R₂ represents an alkyl group that may be substituted, or a heterocycloalkyl that forms a ring via N to which R₂ is bonded, and that may be substituted.

The term "alkyl", by itself or as part of another substituent, refers to fully saturated hydrocarbon represented by the formula CₓH₂ₓ₊₁, where x is a number greater than or equal to 1. In general, the alkyl group of the present invention contains 1 to 20 carbon atoms, preferably 1-10 carbon atoms, and more preferably 1-4 carbon atoms. The alkyl group may be linear or branched, and may be substituted. When a subscript is used herein after a carbon atom, the subscript indicates the number of carbon atoms that the specified group may contain. Thus, for example, C₁₋₄ alkyl means an alkyl having 1 to 4 carbon atoms. Examples of the alkyl group include methyl, ethyl, n-propyl, i-propyl, butyl and its isomers (e.g., n-butyl, i-butyl, and t-butyl); pentyl and its isomers, hexyl and its isomers, heptyl and its isomers, octyl and its isomers, nonyl and its isomers; and decyl and its isomers. C₁-C₆ alkyl includes all linear, branched, and cyclic alkyl groups having 1 to 6 carbon atoms, and accordingly includes methyl, ethyl, n-propyl, i-propyl, butyl and its isomers (e.g., n-butyl, i-butyl, and t-butyl); pentyl and its isomers, hexyl and its isomers, cyclopentyl, 2-, 3-, or 4-methylcyclopentyl, cyclopentylmethylene, and cyclohexyl.

The term "alkoxy", by itself or as part of another substituent, refers to a group consisting of an oxygen atom bonded to a linear or branched alkyl group, a cycloalkyl group, an aralkyl, or a cycloalkylalkyl group, each of which may be substituted. Non-limiting examples of suitable alkoxy groups include methoxy, ethoxy, n-propoxy, isopropoxy, n-butoxy, isobutoxy, sec-butoxy, tert-butoxy, hexanoxy, and the like.

The term "heterocycloalkyl", by itself or as part of another substituent, means a cyclic alkyl group containing at least two different elements (preferably, carbon and nitrogen) in the ring, that is, a monovalent, saturated, or unsaturated hydrocarbyl group having one, two, or three cyclic structures. The term "cycloalkyl" encompasses all saturated or partially saturated (i.e., containing one or two double bonds) hydrocarbon groups containing one to three rings, including monocyclic, bicyclic, and polycyclic alkyl groups. The cycloalkyl group may contain three or more carbon atoms in the ring, and generally contains 3 to 15 atoms according to the present invention. Additional rings in the polycyclic cycloalkyl may be fused, crosslinked, and/or bonded via one or more spiro atoms.

The term "aryl" refers to a polyunsaturated aromatic hydrocarbyl group typically containing 6 to 10 atoms, which may be monocyclic (i.e., phenyl) or contain multiple aromatic rings that are either fused together (e.g., naphthalene or anthracene) or covalently bonded, wherein at least one ring is aromatic. The at least one aromatic ring may optionally include one to three additional rings (any of cycloalkyl, heterocyclyl, or heteroaryl) fused thereto. The term "aryl" is also intended to encompass partially hydrogenated derivatives of the carbon ring systems listed herein. Non-limiting examples of aryl include phenyl, biphenylyl, 5- or 6-tetralinyl, 1-, 2-, 3-, 4-, 5-, 6-, 7-, or 8-azulenyl, 1- or 2-naphthyl, 1-, 2-, or 3-indenyl, 1-, 2-, or 9-anthryl, 1-, 2-, 3-, 4-, or 5-acenaphthylenyl, 3-, 4-, or 5-acenaphthenyl, 1-, 2-, 3-, 4-, or 10-phenanthryl, 1- or 2-pentalenyl, 1-, 2-, 3-, or 4-fluorenyl, 4- or 5-indanyl, 5-, 6-, 7-, or 8-tetrahydronaphthyl, 1,2,3,4-tetrahydronaphthyl, 1,4-dihydronaphthyl, dibenzola,dlcycloheptenyl, and 1-, 2-, 3-, 4-, or 5-pyrenyl. The aryl ring may be substituted with one or more substituents.

The term "halo" or "halogen" is a generic term for fluoro, chloro, bromo, or iodo.

The phrase "may be substituted" means that a specified group may be substituted at any available bonding site with one or more substituents (e.g., one to four substituents, such as one, two, three, or four substituents, or one or two substituents). Non-limiting examples of such substituents include alkyl, halo, hydroxyl, carbonyl, nitro, amino, oxime, imino, azide, hydrazino, cyano, aryl, heteroaryl, cycloalkyl, acyl, alkylamino, alkoxy, thiol, alkylthio, carboxy, carboxyalkyl ester, acylamino, alkyl ester, carbamate, thioamide, urea, sulfonamide, guanidino, and the like.

Preferred examples of the E-64 and/or analogs thereof of the present invention include the compounds represented by the following formulae (II) to (VII).

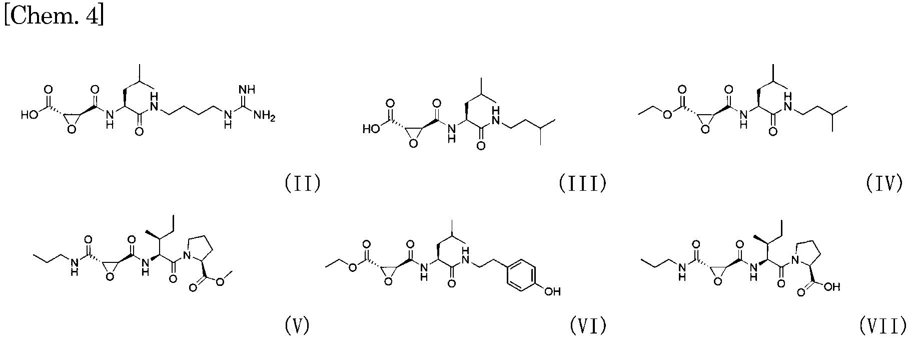

Examples of the calpain inhibitors of the present invention include dazcapistat, alicapistat, TNCA (benzo[b]thiophene-2-carboxylic acid), and the like. The structures of dazcapistat, alicapistat, and TNCA are shown below, respectively.

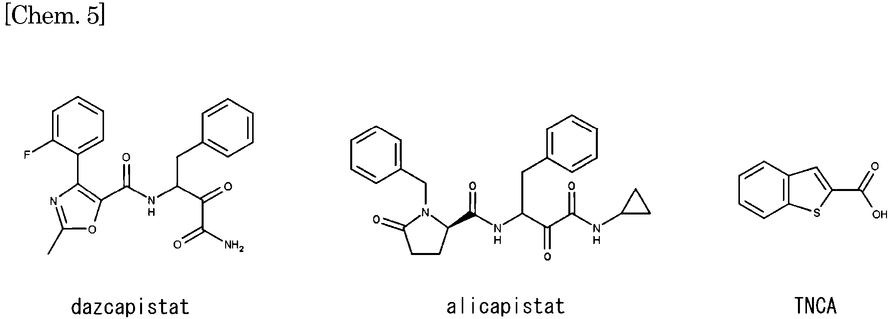

### Examples

Hereinafter, the present invention will be described in detail by way of examples to facilitate a better understanding of the present invention. However, it goes without saying that these examples are not intended to limit the scope of the present invention.

### Example 1

### Quantification of Honey Bee Viruses Using MinION

### (Target Honey Bees)

Honey bee populations from a total of 14 colonies (honey bee populations Nos. 1 to 14), including both colonies critically weakened by mites and healthy colonies, were collected from 13 apiaries in Japan and used as the study subjects.

### (Sample Preparation)

The midgut of honey bees was collected, and total RNA was extracted using ISOGEN II (manufactured by Nippon Gene Co., Ltd.). *E. coli* Poly(A) Polymerase (manufactured by New England BioLabs Japan, Inc. (NEB)) was allowed to act on 30 µg of the RNA to add polyA to the RNA. Subsequently, the polyA-added RNA was purified using Oligotex^{™}-dT30 <Super> mRNA Purification Kit (manufactured by Takara Bio Inc.). Furthermore, excess rRNA was removed using NEBNext rRNA Depletion Kit v2 (Human/Mouse/Rat) with RNA Sample Purification Beads (manufactured by NEB) to obtain sample RNA containing viral RNA. FIG. 1 shows a schematic diagram of the sample preparation procedure for MinION analysis.

### (MinION Analysis Method)

The prepared RNA was used to prepare a library using Direct cDNA Sequencing Kit (manufactured by Oxford Nanopore Technologies plc.) and Native Barcoding Expansion 1-12 (PCR-free) (manufactured by Oxford Nanopore Technologies plc.), and the RNA, converted into cDNA, was quantified using a MinION flow cell (FLO-MIN106D, manufactured by Oxford Nanopore Technologies plc.). The sequence information of the detected viruses was mapped to viral library information, and quantitative values for each virus were calculated. The quantification results are shown in FIG. 2 as a heatmap.

As shown in FIG. 2, Israeli acute paralysis virus (IAPV) and deformed wing virus (DWV) were found to be the predominant viruses.

### Example 2

### RT-PCR-Based Quantification of Honey Bee Viruses

### (Sample Preparation and RT-PCR Analysis Method)

The midgut of honey bees from the honey bee populations Nos. 1 to 14 was collected, and total RNA was extracted using ISOGEN II (manufactured by Nippon Gene Co., Ltd.). Viruses present in the midgut RNA of honey bees were quantified from the total RNA using One Step PrimeScript^{™} III RT-qPCR Mix (manufactured by Takara Bio Inc.) along with the probes and primers shown in Table 1. For quantification, the copy numbers of viruses copresent in the midgut RNA of honey bees were calculated based on the correlation between virus copy numbers and CT values obtained by RT-PCR using DNA fragments derived from RNA virus sequences. FIG. 3 shows the results. The base sequences (DNA) of positive control viruses are shown in Table 2.

As shown in FIG. 3, high levels of virus detection were observed in the honey bee populations Nos. 1, 2, 3, and 5, which derived from the colonies critically weakened by mites. Among these, in the virus-infected groups Nos. 2, 3, and 5, IAPV and DWV were predominant, with IAPV detected at extremely high levels, at least 10 times higher than those of DWV.

**[Table 1]**

| Sequences of primers and probes | | | |
|---|---|---|---|
| Pathogen | Probe (5' -> 3') | Primer (5' -> 3') | |
| | FAM ---- ZEN ----IBFQ(Iowa Black FQ) | | |
| acute bee paralysis virus (ABPV) | ATAGTTAAAACAGCTTTTCACACTGG | CATATTGGCGAGCCACTATG | SEQ ID NO: 10 |
| | SEQ ID NO: 1 | CTACCAGGTTCAAAGAAAATTTC | SEQ ID NO: 11 |
| black queen cell virus (BQCV) | TTTCCATCTTTATCGGTACGCCGCC | GGTGCGGGAGATGATATGGA | SEQ ID NO: 12 |
| | SEQ ID NO: 2 | GCCGTCTGAGATGCATGAATAC | SEQ ID NO: 13 |
| chronic bee paralysis virus (CBPV) | TCAAGAACGAGACCACCGCCAAGTTC | CGCAAGTACGCCTTGATAAAGAAC | SEQ ID NO: 14 |
| | SEQ ID NO: 3 | ACTACTAGAAACTCGTCGCTTCG | SEQ ID NO: 15 |
| deformed wing virus (DWV) | CCTTGACCAGTAGACACAGCATC | GCGGCTAAGATTGTAAATTG | SEQ ID NO: 16 |
| | SEQ ID NO: 4 | GTGACTAGCATAACCATGATTA | SEQ ID NO: 17 |
| sacbrood virus (SBV) | TGATGA+GA+GTG+GACGAAGA | AACGTCCACTACACCGAAATGTC | SEQ ID NO: 18 |
| | SEQ ID NO: 5 | ACACTGCGCGTCTAACATTCC | SEQ ID NO: 19 |
| Varroa destructor virus-1(VDV-1) | AGGTACCCATATCAAGAATACCAGTTGTCC | CGCGTGTCGTTCCTGATT | SEQ ID NO: 20 |
| | SEQ ID NO: 6 | CACTCATACGTAGTGGAGCAATTA | SEQ ID NO: 21 |
| Israeli acute bee paralysis virus(IAPV) | TTTGCGGCAATCCAGCCGTGAAAC | CGAACTTGGTGACTTGAAGG | SEQ ID NO: 22 |
| | SEQ ID NO: 7 | CATCAGTCGTCTTCCAGGTA | SEQ ID NO: 23 |
| kashmir bee virus (KBV) | CTGGATGGGGATATTCCCT | ATCAGAATCAAGTGATGCCTTATCAG | SEQ ID NO: 24 |
| | SEQ ID NO: 8 | GCAGATGGAAATACATCTCGCAAGTC | SEQ ID NO: 25 |
| Lake sinai virus (LSV) | TGCGGACCTCATTTCTTCATGTC | CATCCCAAGAGAACCACTYAC (Y:C or T) | SEQ ID NO: 26 |
| | SEQ ID NO: 9 | CGMGTGTGCATGGAAGAGAG (M:A or C) | SEQ ID NO: 27 |

**[Table 2]**

| | |
|---|---|
| SEQ ID NO: 28 | |
| ABPV | ATCCTGCCGTCAAACATGTGCTGTCTTGGAATACAACGGATAAAGTTCAAGTACCAATAAGTAATGGAGATGACTGGGGAGGAGTGATACCTGTTG GTATGCCATGCTATTCTAAGATAATACGGAAAACAGAGAATGATACAACACAAACTAACACTGAAGTTTTGGATCCTGCCCCTTGTGAATATGTTT GCAATATGTTTTCATATTGGCGAGCCACTATGTGCTATCGTATAGCTATAGTTAAAACAGCTTTTCACACTGGCAGGTTAGAAATTTTCTTTGAACC TGGTAGGATTCCGATAATGACGACGAAAGACAATATTTCCCCGGACTTGACTCAATTAGATGGAACCAAAGCGCCCTCCGACAACAATTATAAATA CATCTTAGATTTGACTAATGATACGGAGATCACCATAAGGGTACCTTTTGTTTCAAACAAAATGTTCATGAAATCTACGGGAATTTATGGTGGAAA TTCTGAAAACAATTGGGAT |
| SEQ ID NO: 29 | |
| BQCV | TGATGTCGCCCCTACTACTCATATCAGTACTCCTTTGGCAATAGAACAAATACCCATAAAGGGAGTCGCAGAGTTCCAAATACCGTACTATGCTCC ATGTTTGTCATCTTCGTTTAGAGCGAATTCGGAAACATTCTACTATAGTTCAGGTCGGAATAATCTCGATATAGCCACATCACCTCCTTCCATCAAT CGCTATTATGCGGTAGGTGCGGGAGATGATATGGACTTTTCCATCTTTATCGGTACGCCGCCCTGTATTCATGCATCTCAGACGGCTCAGTTTACC AAGATAAAACAAGGTAAAGTGTATGATTTGAGGTATGATCAGTATGACCCTTTCAGGGAAGTCCAGGACGGTACGGCGTTCCTCAATGCTCGTAGT ATTGAGGATAGCGATTTGTTGTGAGCTCCTTTAGAGGGAGGGCTCACTTTATCTATTGCTTAAATCGGTAAGCCACAAATTTTTTCAAGTGTCATG AGTTTCTTCTCGGTTCTTC |
| SEQ ID NO: 30 | |
| CBPV | CATCAAACGGGAGTCCGGATCCCCGAGACACTCCGTTACGCCAGTGCGTCGAAGAATTCTTAATGACACCTCAGATGAGCTGGTTGAAGAAGGTT CATTCTCCTGTAGATTTCTCGATATGGGTCAAACGTTACCCCGAATGGCGCCAACGGCAGCTCATCGCCGCACGAAATCGCGTCCTCAGCGACCCA GAACCTCGCCGCAAGTACGCCTTGATAAAGAACTTTGTCAAGAACGAGACCACCGCCAAGTTCGTGGACCCAAGAAACATCAGTCCTCGAAGCGA CGAGTTTCTAGTAGTGGTGGGACCTTACATCAGCGCCATCGAACACGCCGCGGTTCATTGCCCATTCCTCATCAAGGGGCTGACACCGAAGAAGA GATGTGATAAGTTATCCTGGTTAACAGAGTATGAGCGATTTCTTGAGATCGACTTCGCTCGTTTCGATCAGACTCTGATGAAAGATCTCTTGCGTA TTGTCGAATTAAGATTTTTACTC |
| SEQ ID NO: 31 | |
| DWV | CCCTTGTTTACCACAAGGGTTAGCGTTAGACATTGGTTCTGCTGGCTCTCCACATGAAATATTTAATAGATGTCGTGATGGTATTATACCATTAATT GCATCTGGATATAGATTTTATAGAGGCGATTTGCGTTATAAGATCGTTTTTCCAAGTAATGTTAATAGCAACATTTGGGTACAACATCGACCGGAT CGTAGACTGGAAGGATGGTCTGCAGCTAAGATTGTAAATTGTGATGCTGTGTCTACTGGTCAAGGAGTATATAATCATGGTTATGCTAGTCACATT CAAATCACGCGTGTAAATAACGTTATAGAATTGGAAGTTCCATTTTATAATGCTACTTGTTATAATTATTTACAGGCGTTTAATGCGTCTAGCGCTG CATCTAGTTATGCAGTATCTTTAGGAGAAATATCGGTTGGTTTTCAAGCTACAAGTGATGATATTGCATCTATTGTTAACAAACCTGTTACTATTTA TTATAGTATTGGAGATG |
| SEQ ID NO: 32 | |
| SBV | GTACCAACCGATTCCTCAGTTGTGGAGGAATCAGAAGAAATCAGTCTTCTGAATATAGTTCACGCGCCAGAACCTATAAAACCAAGTTGGAGGCGC GTAATAGCGGAGTGGAGAGATTATCTACAATCCTTACCTCTTGTAAGAAGACATTTGATACAGTGGACTCTTATACCGATTTGTTTAATGGTTGGG TTTCTGGTATGTATGTTGACAAGAACGTCCACTACACCGAAATGTCCAGTGATGAGAGTGGACGAAGAATCTGGAATGTTAGACGCGCAGTGTCAA TTAAGACCGCAGAAGGAACTATAGTATGGCGAAAAGTTATTACCTCTTACAGTTGCAAGGTGGCTTCGGAACTAGCTGCTAAGAGTATACTGGTGC AATTTGTAGGTCCAATAAGGACCCAGAGTGATGAGGTACCCTCGAAAGAATCTATTCAGGGGGACGCTACACAGCAAAGTTCCAAGGAAGAAAAT ACAATAATTACACGAGACCAG |
| SEQ ID NO: 33 | |
| VDV1 | TCGAGTGCAGATTAACTCGAATAAATTCCAGGTTGGTCAATTACAGGCAACTTGGTACTATTCGGATCATGAAAATTTGAATATCCAGACGAAGCG AAGTGTGTATGGTTTTTCGCATATGGATCATGCTTTGATTAGCGCATCAGCGAGTAATGAAGCAAAATTAGTGATACCTTTTAAACACGTATATCC ATTCTTACCAACGCGTGTCGTTCCTGATTGGACAACTGGTATTCTTGATATGGGTACCTTAAATATTCGTGTAATTGCTCCACTACGTATGAGTGC GACGGGACCAACCACTTGTAATGTTGTAGTATTTATTAAGTTAAATAATAGTGAATTCACTGGCACTTCTTCTGGTAAGTTTTACGCGAATCAAATC AGGGCAAAACCTGAAATGGACCGTGTGTTAAATTTGGCAGAAGGATTACTAAATAATACCGTAGGTGGTTGTAATATGGATAATCCGTCATATCAG CAATCTCCGCGTCATTTTG |
| SEQ ID NO: 34 | |
| IAPV | GAAATTGCAAAGCCTGTAACAACTGCTGTAAAGTGGTTTGCAGATGTTGTCGGAACTGTGGCAGCTATTTTTGGTTGGTCCAAACCTCGAAATCAACAACAGGTATGCC CATTACAAAATGTGCCTGCATGGGGATATTCTCTCTATAAAGGCATAGATATGAGCGTACCATTGGCTTATGACCCTAATAACGAACTTGGTGACTTGAAGGATGTATT TCCTTCTGCGGTTGATGAAATGGCTATAGGGTATGTTTGCGGCAATCCAGCCGTGAAACATGTTCTTACCTGGAAGACGACTGATGCAATTCAGAAACCAATAGCGAAC GGAGATGATTGGGGTGGAGTTATACCAGTGGGAATGCCTTGTTATTCTAAATCCATTAGAACTACAAGTATTTCAGAAACGGAAAATCGTGAAACTGAAGTCATGGATG CTGCTCCATGTGAATATGTTGCTAACATGTTCTCATATTGGCGTGCAACTATGTGTTATAGGAT |
| SEQ ID NO: 35 | |
| KBV | TTGGACCAATTTCTGAAGTGGCTTCAGGGGTTAAGACGGCTGCAAACGGCATCGAGCGCATTCCAG TGCTAGGTGAAATTGCAAAGCCGGTAACCGCTGCAGTTAAATGGTTTGCAGACATTGTAGGAGGTGTAGCAGCCATATTTGGGTGGTCCAAACCCCGAAATC AGAATCAAGTGATGCCTTATCAGAATGTGCCTGGATGGGGATATTCCCTGTACAAAGGCATTGACATGAGCGTGCCACTTGCTTATGACCCCAATAATGAAT TAGGAGACTTGCGAGATGTATTTCCATCTGCTGTAGACGAAATGGCTATTGGGTATGTTTGTGGCAATCCAGCTATAAAACATGTCCTCACCTGGAATACAA CAGATGTAGTACAGACCCCAATAAGTAACGGAGATGATTGGGGAGGAGTAATACCAGTGGGAATGCCTTGCTATTCTAAGACCATTAGATCAGTTAAAGGC GACACTTCAACTTTAAAAACCGAGGTG |
| SEQ ID NO: 36 | |
| LSV | GACTCCTCTACGTCGGGATCAGTCTTATTATTCGACGACACTGCCGACCGAGTTCATGAGGATGACTCGTCCGCGAGATCGACGCCGTGTCGAGGTTCTCGA TTACGTGCTAACAAGAGCTACCCAGGGCGCAAGTCCCGTCGACCTGCGGACCATAACTCCGGCAGTGACAACCAAATCGACGTTCCTCATCCCAAGAGAAC CACTCACCCCCTGTGCCCCGACCCGACTGCCTCGTGCGGACCTCATTTCTTCATGTCAGTGTGTGAGTCAAATGAGTCAGTACCTACTCTCTTCCATGCACAC TCGGTGGGAGGTGAAGATATCACCCATGTTGTTGATCCGGCTCTGGGAGCGTCTATATCGCAACGGTTTAGCGCTTCGCAGTTACGCCTCCTTGGCTGGTCTA TCGACGGAATTTTCAATACTTTATCTGGCGCCGCCACCTCGTCATTCGTCGAGTCGACGTTGTTGTGCTTATCACGATTTATGCGTGAAG |

### Example 3

RNA-seq Analysis of Honey Bee Biological Factors and Quantification of Each Factor

### (Sample Preparation and RT- PCR Analysis Method)

The midgut was collected from individual honey bees in the virus-infected groups Nos. 1, 2, 3, and 5, as well as from individual honey bees in the virus-uninfected groups Nos. 6, 7, 9, and 14, and total RNA was extracted using ISOGEN II (manufactured by Nippon Gene Co., Ltd.). mRNA was purified using oligo(dT), and cDNA was synthesized using random primers. Subsequently, sequencing libraries were prepared. Sequencing analysis (DNBSEQ-G400, 100 bp paired-end analysis) was performed using the resulting libraries. Data analysis was performed based on the sequence information obtained as a result of the above analysis, and mRNA levels in the midgut of honey bees from the honey bee populations Nos. 1, 2, 3, 5, 6, 7, 9, and 14 were quantified. A list was compiled showing the number of factors whose gene expression was increased or decreased in the virus-infected groups Nos. 1, 2, 3, and 5 compared to that of the virus-uninfected groups Nos. 6, 7, 9, and 14. The list of differentially expressed genes is shown in Table 3. FIG. 4 shows the quantification results for virus response factors and antimicrobial peptides, and FIG. 5 shows the quantification results for intestinal tract repair factors. The quantitative values of each factor shown in the figures were calculated based on the results of the quantitative numerical values obtained through RNA-seq analysis.

As shown in Table 3, decreases in the expression of immune factors and metabolism-related factors and increases in the expression of apoptosis-related factors were observed *in vivo* in virus-infected honey bees. These findings revealed that IAPV or DWV induces individual honey bee mortality by reducing immune functions and metabolic functions and by promoting cell death and tissue degradation, through the differential expression of these factors. Furthermore, based on the analyses including FIG. 4, FIG. 5, and the histochemical analysis described in Example 5 below, it was also revealed that these viruses formed pores in the intestinal tract, thereby allowing bacteria to enter the body, inducing sepsis, and consequently leading to reduced immune and metabolic functions, cell death, and tissue degradation.

**[Table 3]**

| List of differentially expressed genes | | |
|---|---|---|
| | up | down |
| Immune | 9 | 18 |
| Glycolysis | 0 | 4 |
| TCA cycle | 0 | 4 |
| Electron transport chain | 0 | 26 |
| Apoptosis | 13(7) | 8 (2) |
| autophagy | 9 | 13 |
| ubiquitin | 13 | 28 |
| Translation | 14 | 138 |
| RNA processing | 8 | 29 |
| nuclease | 1 | 27 |
| DNA repair | 3 | 18 |
| replication | 7 | 33 |
| Actin | 28 | 45 |
| Microtubule | 28 | 78 |
| intestinal tract damage response | 3 | - |
| virus-infected | 2 | - |

| | | |
|---|---|---|
| No change in expression of CCR4-Not | | |

### Example 4

### RNAi Tests for Honey Bee Viruses

### (dsRNA Preparation Method and RNAi Testing Method)

Israeli acute paralysis virus (IAPV) was cloned using two sets of primers (IAPV Cloning primers 1-F and 1-R (SEQ ID NOs: 37 and 38) and IAPV Cloning primers 2-F and 2-R (SEQ ID NOs: 39 and 40)). Deformed wing virus (DWV) was cloned using a single set of primers (DWV Cloning primers F and R (SEQ ID NOs: 41 and 42)). Subsequently, based on DNA fragments, IAPV dsRNA (a mixture of IAPV dsRNA 1 and IAPV dsRNA 2) and DWV dsRNA were synthesized using the T7 RiboMAX^{™} Express Large Scale RNA Production System (manufactured by Promega Corporation) along with primers for each virus, that is, IAPV T7 primers 1-F and 1-R (SEQ ID NOs: 43 and 44) and IAPV T7 primers 2-F and 2-R (SEQ ID NOs: 45 and 46), and DWV T7 primers F and R (SEQ ID NOs: 47 and 48). For GFP, GFP dsRNA was synthesized using a GFP-linked vector (pEGFP-C3) and primers (GFP T7 primers F and R (SEQ ID NOs: 49 and 50)). Each dsRNA was dissolved in a 50% sucrose solution at a concentration of 100 µg/ml and administered to honey bees from the virus-infected honey bee population No. 5. The 50% sucrose solution containing dsRNA was replaced daily, and changes in the number of individual honey bees were measured each day. A 50% sucrose solution without dsRNA was administered to a control group. Rearing was conducted using an aseptic *in vivo* agent evaluation rearing method.

### (Aseptic in vivo Agent Evaluation Rearing Method)

A new aseptic *in vivo* agent evaluation rearing method for honey bees was established. Incubators and rearing boxes were sterilized with 70% ethanol after cleaning. Each hive box was populated with 30 to 35 honey bees. The honey bees were fed with a 50% sucrose solution (aseptically dissolved in sterile water) and a 50% sucrose solution containing pollen dissolved therein. The rearing temperature was maintained at 30°C. The 50% sucrose solutions were replaced daily, and the number of individual honey bees was measured at each replacement. Agents were administered dissolved in the 50% sucrose solutions. At the time when the viability had decreased to 50% or less of the initial level, total RNA was collected from the surviving individuals, and histochemical analyses were performed on the midgut and other tissues. Pharmacological analyses were conducted using the collected RNA, tissues, and blood.

As shown in FIG. 6, RNAi tests showed that RNAi for IAPV suppressed individual mortality and sepsis in honey bees following mite infestation, whereas RNAi for DWV had no such effect. These results revealed that IAPV is the pathogen responsible for the mass mortality of honey bees.

### Example 5

### Analysis of Intestinal Tract Regeneration in Midgut of Honey Bees

### (Sample Preparation Method and Detection Method)

The honey bee midgut was dissected and subsequently fixed in 4% paraformaldehyde for 30 minutes. After fixation, washing was performed three times with PBS containing 0.2% Triton X-100 (hereinafter also referred to as "PBX") at room temperature for 20 minutes each. Blocking was then performed by allowing a reaction with 2% BSA/PBX at room temperature for 1 hour. Next, the resulting sample was reacted with 0.5% BSA/PBX containing Phospho-Histone H3 (pH3) Antibody (manufactured by Cell Signaling Technology, Inc.) at 4°C for 8 hours. After washing was performed three times with PBX at room temperature for 20 minutes each, the sample was reacted with 0.5% BSA/PBX containing a secondary antibody at 4°C for 8 hours. Subsequently, washing was performed three times with PBX at room temperature for 20 minutes each. The sample was then mounted on a glass slide, and the number of pH3 antibody-positive cells was counted using a fluorescence microscope. Microscopic images were observed using a confocal laser microscope. In addition, the dsRNA of IAPV, DWV, or GFP prepared in Example 4 was administered, and the number of pH3 antibody-positive cells in the midgut of each individual was counted on day 7 of rearing.

FIG. 7 shows microscopic images from the midgut of the virus-infected group (honey bee population No. 5) and the virus-uninfected group. FIG. 8 shows the number of pH3 antibody-positive cells in the midgut on day 7 of rearing for each group to which the dsRNA of IAPV, DWV, or GFP was administered. pH3 antibody enables the detection of intestinal tract regenerative cells. More specifically, intestinal tract regeneration occurred in response to damage in the intestinal tract, and this result means that the intestinal tract had been damaged. As shown in FIG. 7, intestinal tract regenerative cells were detected in the virus-infected group, confirming midgut damage and suggesting the involvement of protease from the infecting virus. As shown in FIG. 8, dsRNA of IAPV was confirmed to suppress midgut damage.

### Example 6

### Suppression of Individual Mortality of Honey Bees by E-64 Administration

### (Experimental Method and Administration Method)

E-64 ([(2S,3S)-3-carboxyoxirane-2-carbonyl]-L-leucine (4-guanidinobutyl)amide hemihydrate, manufactured by PEPTIDE INSTITUTE, INC.) was dissolved in a 50% sucrose solution at concentrations of 200 µM, 100 µM, and 50 µM. The resulting solutions were administered to honey bees in the honey bee populations Nos. 2 and 5, in which viral infection had been observed. Only 50% sucrose was administered to a control group (None). The E-64-containing solutions were replaced daily, and changes in the number of individual honey bees were measured at each replacement. FIGS. 9 and 10 show the results. Rearing was conducted using the aseptic *in vivo* agent evaluation rearing method, which has been described above.

Since the protease of IAPV is a cysteine protease called a 3C-like protease, administration of E-64, which is a cysteine protease inhibitor, to two lines derived from the colonies critically weakened by mites suppressed individual honey bee mortality due to mite infestation, as shown in FIGS. 9 and 10. Thus, it was revealed that the viral protease was responsible for the proliferation of the pathogenic virus and the damage to the intestinal tract. These results indicate that E-64 can be utilized as an antiviral agent to suppress mass mortality of honey bees by inhibiting the protease of IAPV.

### Example 7

### BPB Administration Test

### (Experimental Method)

Virus-infected and uninfected honey bees were reared for 10 days while receiving 50% sucrose. During the rearing period, Bromophenol Blue (BPB) (FUJIFILM Wako Pure Chemical Corporation) was dissolved in a 50% sucrose solution at a concentration of 10 mg/ml and administered to the virus-infected and uninfected honey bees for 24 hours. When the viability of the virus-infected group had decreased to approximately 50%, relative to the number of individual honey bees before rearing set to 100%, BPB was administered to each group of honey bees. After BPB administration, blood was collected from honey bees (≥ 30 individuals), and the concentration of BPB in the blood was measured by absorbance at 500 nm using a microvolume spectrophotometer, NanoDrop One (ThermoFisher Scientific).

When measuring blood at an absorbance of 500 nm, substances other than BPB may be detected. Therefore, as a control, blood was also collected from honey bees to which sucrose was administered, and the measurement was performed. As a result, in the virus-infected group, the concentration of BPB in the blood increased following administration of BPB compared to administration of sucrose, whereas no such increase was observed in the uninfected group following BPB administration (FIG. 11). In uninfected honey bees, the intestinal tract is normal, and therefore BPB is not absorbed into the body and is not detectable in the blood. In contrast, in honey bees having intestinal tract damage caused by viral infection, BPB is absorbed into the body and becomes detectable in the blood. Based on the results of the present Example, it was confirmed that the virus-infected honey bees exhibited intestinal tract damage.

### Example 8

### Measurement of Viable Bacterial Count in Honey Bee Blood

### (Experimental Method)

Blood was collected from virus-infected honey bees (≥ 100 individuals) and virus-uninfected honey bees (≥ 100 individuals), and the viable bacterial count in the blood was measured using MRS medium (Biokar Diagnostics). The collected blood was plated onto MRS medium, and the viable bacterial count was measured after 24 hours.

Normally, no bacteria are present in the blood of honey bees. Although blood collection was performed with sufficient disinfection, a small number of bacteria were inevitably detected even in the blood of virus-uninfected honey bees, because the honey bees utilized were reared outdoors. However, the blood of virus-infected honey bees contained approximately ten times more bacteria compared to that in the blood of virus-uninfected honey bees (FIG. 12). These results suggest that viral infection caused damage to the intestinal tract of honey bees, thereby allowing intestinal bacteria to translocate into the blood and become detectable in the blood. The detected bacteria are believed to non-specifically degrade honey bee-derived mRNA, ultimately leading to the death of the honey bees.

### Example 9

### Confirmation Experiment on Recovery from Intestinal Tract Damage in Honey Bees by Administration of E-64

### (Experimental Method)

Virus-infected honey bees were divided into a control group (None) to which 50% sucrose was administered, and an administered group (E-64) to which a solution of E-64 (PEPTIDE INSTITUTE, INC.) dissolved at 100 µM in 50% sucrose was administered. The honey bees were reared for 10 days. During the rearing period, Bromophenol Blue (BPB) (FUJIFILM Wako Pure Chemical Corporation) was dissolved in a 50% sucrose solution at a concentration of 10 mg/ml and administered to each group of honey bees for 24 hours. When the viability of the control group had decreased to approximately 50%, relative to the number of individual honey bees before rearing set to 100%, BPB was administered to each group of honey bees. After BPB administration, blood was collected from honey bees (≥ 30 individuals), and the concentration of BPB in the blood was measured by absorbance at 500 nm using a microvolume spectrophotometer, NanoDrop One (ThermoFisher Scientific).

As a result, in the control group, the concentration of BPB in the blood increased following administration of BPB compared to administration of sucrose. In contrast, administration of E-64 reduced the concentration of BPB in the blood after administration of BPB to a level comparable to the values observed in individual honey bees from the virus-uninfected group after BPB administration in Example 7 (FIG. 11) (FIG. 13). These results indicate that intestinal tract damage in honey bees caused by the virus was restored to normal levels by the administration of E-64.

### Example 10

### Protein Expression of IAPV Protease

### (Experimental Method)

RNA was extracted from the midgut of virus-infected honey bees using ISOGEN II (Nippon Gene Co., Ltd.), and the extracted RNA was reverse-transcripted using PrimeScript RT reagent Kit (Takara) to obtain midgut-derived cDNA. Subsequently, the midgut-derived cDNA was subjected to PCR reaction (30 cycles of 98°C for 30 seconds, 60°C for 30 seconds, and 68°C for 2 minutes) using KOD FX Neo (TOYOBO Co., Ltd.) with IAPV Protease Primer-F1 (SEQ ID NO: 59) and IAPV Protease Primer-R (SEQ ID NO: 60). As a result, a cDNA fragment of IAPV protease was obtained, consisting of the base sequence of SEQ ID NO: 57 encoding the amino acid sequence of SEQ ID NO: 58. The cDNAfragment of the IAPV protease was subjected to restriction enzyme treatment with BamHI and HindIII. The expression vector pET21a (Merck) was also subjected to restriction enzyme treatment with BamHI and HindIII. The restriction enzyme-treated cDNA fragment of the IAPV protease and the restriction enzyme-treated pET21a DNA fragment were subjected to a ligation reaction at 16°C for 1 hour using Ligation High Ver. 2 (TOYOBO Co., Ltd.) to join the vector and the cDNA fragment, and the ligation product was used to transform *E. coli* Colony PCR was performed, and an expression vector containing the cDNA fragment of the IAPV protease was obtained. *E. coli* Rosseta-gami B (Merck) was transformed with the expression vector of the IAPV protease. A preculture was prepared in 50 ml of LB medium containing ampicillin, followed by subculturing in 500 ml of LB medium containing ampicillin. Then, the culture was grown until the absorbance at 600 nm reached 0.5, at which point isopropyl β-D-thiogalactopyranoside (IPTG) was added to a final concentration of 1 mM. The culture was then incubated at 15°C for 24 hours. The culture was centrifuged (8000 rpm, 10 minutes, 4°C) to collect *E. coli* cells. The collected *E. coli* cells were dissolved in 10 ml of a sonication buffer (50 mM Tris-HCl, pH 8.0; 50 mM NaCl; 1 mM EDTA; 1 mM DTT), and disrupted by ultrasonic disruption (sonication). The disruption solution was centrifuged (12000 rpm, 10 minutes, 4°C) to obtain a supernatant of this bacterial cell disruption solution. The IAPV protease was then purified from the resulting supernatant to yield a purified protein solution. Purification was carried out by cation exchange chromatography using HiPrep SP XL 16/10 (Cytiva) with a 20 mM Tris-HCl (pH 7.5) buffer and a 20 mM Tris-HCl (pH 7.5) buffer containing 1 M NaCl, and by gel filtration chromatography using HiLoad 16/600 Superdex 200 (Cytiva) with a 50 mM phosphate buffer (pH 7.0) containing 0.15 M NaCl.

### Example 11

### Measurement of IAPV Protease Activity Using Purified Enzyme

### (Experimental Method)

As a substrate for the measurement of IAPV protease activity, either Dnp-PNDIV DVTMQK(Nma)-NH₂ or Nma-TMQMWKDQVAK(Dnp)-NH₂, in which a peptide was linked to a fluorescent substrate, was synthesized and used. When the activity measurement substrate is cleaved by the protease, it emits fluorescence. The activity measurement substrate was prepared at a concentration of 20 µM in an assay buffer (50 mM citric acid buffer, pH 6.0; 150 mM NaCl; 1 mM EDTA; 1 mM DTT). The purified protein solution of the IAPV protease obtained in Example 10 was used as the enzyme solution. 50 µl of the assay buffer containing the activity measurement substrate was mixed with 50 µl of the enzyme solution, and the mixture was reacted at 37°C for 30 minutes in a 96-well black plate, in the presence or absence of various inhibitors listed in Table 4. Fluorescence intensity was then measured using a TECAN fluorescence plate reader (Tecan Japan Co., Ltd.) at an excitation wavelength of 340 nm and a fluorescence emission wavelength of 440 nm. The various inhibitors were prepared at a final concentration of 1 mM and pre-incubated at 37°C for 1 hour prior to the enzymatic reaction.

Table 4 shows the relative activity of the IAPV protease calculated from the fluorescence intensity measured in the presence of the various inhibitors, with the fluorescence intensity measured in the absence of inhibitors ("None" in Table 4) defined as 100% relative activity. As shown in Table 4, E-64 analogs E-64, E-64c, E-64d, CA074 methyl ester (CA-074Me), JPM-OEt, and CA074, as well as calpain inhibitors dazcapistat, alicapistat, and TNCA, all inhibited IAPV protease activity. These results supported a mechanism by which E-64 and its analogs, as well as calpain inhibitors, act on individual honey bee mortality through IAPV protease inhibition.

**[Table 4]**

| Inhibition of IAPV Protease Activity by compounds | |
|---|---|
| | Relative Activity (%) |
| None | 100 |
| E-64 | 5 |
| E-64c | 22 |
| E-64d | 23 |
| CA074 methyl ester | 32 |
| JPM-OEt | 17 |
| CA074 | 4 |
| dazcapistat | 29 |
| alicapistat | 22 |
| TNCA | 71 |

E-64, E-64c, and E-64d used were manufactured by PEPTIDE INSTITUTE, INC.; CA074 methyl ester (CA-074Me), JPM-OEt, CA074, dazcapistat, and alicapistat used were manufactured by MedChemExpress; and TNCA used was manufactured by BLD Pharmatech Ltd.

### Example 12

### Protein Expression and Protease Activity Measurement of IAPV Protease

### (Experimental Method)

Midgut-derived cDNA was obtained from virus-infected honey bees in the same manner as in Example 10 and then subjected to PCR reaction (30 cycles of 98°C for 30 seconds, 60°C for 30 seconds, and 68°C for 2 minutes) using KOD FX Neo (TOYOBO Co., Ltd.) with IAPV Protease Primer-F2 (SEQ ID NO: 61) and IAPV Protease Primer-R (SEQ ID NO: 60). As a result, a cDNA fragment of IAPV protease was obtained, consisting of the base sequence of SEQ ID NO: 57 encoding the amino acid sequence of SEQ ID NO: 58. The cDNA fragment of the IAPV protease was subjected to restriction enzyme treatment with BamHI and HindIII. The expression vector pMAL-c6T (New England Biolabs, Inc.) was also subjected to restriction enzyme treatment with BamHI and HindIII. The restriction enzyme-treated cDNA fragment of the IAPV protease and the restriction enzyme-treated pMAL-c6T DNA fragment were subjected to a ligation reaction at 16°C for 1 hour using Ligation High Ver. 2 (TOYOBO Co., Ltd.) to join the vector and the cDNA fragment, and the ligation product was used to transform *E. coli.* Colony PCR was performed, and an expression vector containing the cDNA fragment of the IAPV protease was obtained. *E. coli* Rosseta-gami B (Merck) was transformed with the expression vector of the IAPV protease. A preculture was prepared in 50 ml of LB medium containing ampicillin, followed by subculturing in 500 ml of LB medium containing ampicillin. Then, the culture was grown until the absorbance at 600 nm reached 0.5, at which point IPTG was added to a final concentration of 1 mM. The culture was then incubated at 15°C for 24 hours. The culture was centrifuged (8000 rpm, 10 minutes, 4°C) to collect *E. coli* cells. The collected *E. coli* cells were dissolved in 10 ml of a sonication buffer (50 mM Tris-HCl, pH 8.0; 50 mM NaCl; 1 mM EDTA; 1 mM DTT), and disrupted by ultrasonic disruption (sonication). The disruption solution was centrifuged (12000 rpm, 10 minutes, 4°C) to obtain a supernatant of this bacterial cell disruption solution. The results of protein analysis by SDS-PAGE showed that the expressed fusion protein of Maltose Binding Protein (MBP)-IAPV protease, expressed using pMAL-c6T, accounted for approximately 40% to 50% of the total protein.

Subsequently, using a crude enzyme solution of the expressed fusion protein of IAPV protease as the enzyme solution, the IAPV protease activity was measured using the same method as in Example 11, in the presence or absence of E-64.

The fluorescence intensity measured in the absence of E-64 was reduced by the addition of E-64. When the fluorescence intensity measured in the absence of E-64 is defined as 100% relative activity, the relative activity of IAPV protease calculated from the fluorescence intensity measured in the presence of E-64 was 9% (FIG. 14). Accordingly, the inhibitory activity of E-64 against IAPV protease was confirmed using the IAPV protease synthesized in an *E*. *coli* protein synthesis system.

### Example 13

### Analysis of Effects of E-64c and E-64d on Individual Honey Bee Mortality

### (Experimental Method)

E-64c (manufactured by PEPTIDE INSTITUTE, INC.) or E-64d (manufactured by PEPTIDE INSTITUTE, INC.) was dissolved in a 50% sucrose solution at a concentration of 100 µM. The resulting solutions were administered to honey bees in the honey bee population No. 2, in which viral infection had been observed. Only 50% sucrose was administered to a control group (None). The solution containing E-64c or E-64d was replaced daily, and changes in the number of individual honey bees were measured at each replacement. Rearing was conducted using the aseptic *in vivo* agent evaluation rearing method, which has been described above.

As a result, the group to which E-64c was administered and the group to which E-64d was administered exhibited higher viability compared to the control group. This confirmed that E-64c and E-64d are each capable of suppressing a honey bee disease that is caused by mite infestation and that leads to individual honey bee mortality (FIG. 15). Based on these results, E-64c and E-64d inhibit the activity of IAPV protease and can be employed as prophylactic and/or therapeutic agents against a honey bee disease that is caused by intestinal tract damage in honey bees and that leads to individual honey bee mortality.

### Industrial Applicability

According to the present invention, screening of compounds using viral protease inhibition as an indicator can provide a key technology for the development of antiviral agents that contribute to the suppression of mass mortality of honey bees. This can be expected to lead to the development of an agent that is effective for the conservation of honey bees and exhibits a high level of safety.

## Claims

1. A method for screening a prophylactic and/or therapeutic agent against a honey bee disease that is caused by intestinal tract damage in honey bees and that leads to individual mortality, comprising using inhibitory activity against a virus-derived protease as an indicator.

2. The screening method according to claim 1, wherein the honey bees are honey bees infected with a virus transmitted by a mite.

3. The screening method according to claim 1 or 2, wherein the protease is a cysteine protease.

4. The screening method according to claim 1 or 2, wherein the virus is Israeli acute paralysis virus (IAPV) or deformed wing virus (DWV).

5. The screening method according to claim 1, further comprising use of individual honey bee mortality and/or honey bee intestinal tract regeneration as an indicator.

6. A prophylactic and/or therapeutic agent against a honey bee disease that is caused by intestinal tract damage in honey bees and that leads to individual honey bee mortality, the agent comprising a substance that inhibits activity of a virus-derived protease.

7. The prophylactic and/or therapeutic agent according to claim 6, wherein the substance that inhibits activity of the virus-derived protease is E-64 and/or an analog thereof represented by the general formula (I) below: 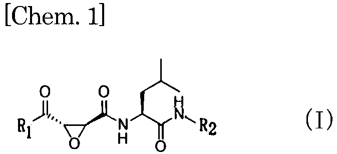 where
R₁ represents OH, an alkoxy group that may be substituted, or an amino group that may be substituted; and
R₂ represents an alkyl group that may be substituted, or a heterocycloalkyl that forms a ring via N to which R₂ is bonded, and that may be substituted.

8. The prophylactic and/or therapeutic agent according to claim 6, wherein the substance that inhibits activity of the virus-derived protease is a calpain inhibitor.
